# EUROPÄISCHE PATENTSCHRIFT

(11) **EP 2 231 584 B1**
(45) Veröffentlichungstag und Bekanntmachung des Hinweises auf die Patenterteilung: **04.09.2013**
(21) Anmeldenummer: 09701997.0
(22) Anmeldetag: 14.01.2009
(51) Int. Cl.: C07C 209/72, C07C 211/36, C08K 5/00, C23F 11/14, C08K 5/18

(54) **VERFAHREN ZUR HERSTELLUNG VON CYCLOALIPHATISCHEN AMINEN**
METHOD FOR THE PRODUCTION OF CYCLOALIPHATIC AMINES
PROCÉDÉ POUR PRODUIRE DES AMINES CYCLOALIPHATIQUES

(30) Priorität: 18.01.2008 EP 08150395
(43) Veröffentlichungstag der Anmeldung: 29.09.2010
(73) Patentinhaber: BASF SE, 67056 Ludwigshafen (DE)
(72) Erfinder: PFEFFINGER, Joachim, 67071 Ludwigshafen (DE); DOELL, Willi, 67259 Beindersheim (DE)
(86) Internationale Anmeldenummer: PCT/EP2009/050341
(87) Internationale Veröffentlichungsnummer: WO 2009/090179

(56) Entgegenhaltungen:
- EP-A1- 1 369 447
- EP-A2- 1 251 119
- DE-A1- 4 404 220
- DE-A1- 19 533 718
- US-A- 5 214 212
- T.S. CARSWELL ET AL: "Cyclohexylamine and Dicyclohexylamine" INDUSTRIAL AND ENGINEERING CHEMISTRY, Bd. 29, Nr. 11, 10. September 1937 (1937-09-10), Seiten 1247-1251, XP002533816

## Beschreibung

Die vorliegende Erfindung betrifft ein Verfahren zur Herstellung von cycloaliphatischen Aminen durch Hydrierung der entsprechenden aromatischen Verbindungen in Gegenwart von Ruthenium (Ru) enthaltenden Katalysatoren, in Gegenwart von suspendierten, anorganischen Zusatzstoffen. Weiterhin betrifft die vorliegende Erfindung die Verwendung der so erhältlichen cycloaliphatischen Amine.

In US 2,606,925 wird die Hydrierung von aromatischen Verbindungen, welche über N-Atome direkt am aromatischen Kern substituiert sind (beispielsweise aromatische Amine oder Nitroverbindungen), zu den entsprechenden cycloaliphatischen Verbindungen beschrieben. Als Katalysatoren werden elementares Ru-Metall, Ru-Oxide, Ru-Salze, Ru-Sulfide und Ru-Sulfate verwendet. Optimale Ergebnisse können entsprechend dieser Schrift erzielt werden, wenn das Ru in fein verteilter Form vorliegt. Eine Methode zur Herstellung dieser Katalysatoren wird jedoch nicht dargelegt.

In US 2,494,563 werden feinverteilte Ru-haltige Katalysatoren zur Hydrierung von aromatischen Diaminen zu den entsprechenden aliphatischen Diaminen eingesetzt.

Eine Methode zur Herstellung sehr kleiner Rutheniumdioxid-Partikel durch Umsetzung von RuCl₃-Lösung mit NaOH in wässriger Phase, Waschung und Trocknung ist in CA-A-860855 beschrieben. Dabei werden Kristallitgrößen kleiner als 50 nm erhalten. Als bevorzugte Verwendung des dabei erhaltenen Pulvers wurde die Herstellung von elektrischen Widerständen angegeben.

DE-OS-2132547 offenbart ein Verfahren zur Hydrierung von aromatischen Verbindungen zu den entsprechenden Cycloaliphaten. Für die Hydrierung wird ein Katalysator verwendet, der auf Oxidhydraten von Ru basiert. Die Herstellung des Katalysators erfolgt durch Fällung aus einer wässrigen Lösung eines Ru-Salzes durch Zugabe von Alkalilauge. Das so erhaltene Ru-Oxidhydrat kann direkt in das Verfahren eingesetzt werden oder vor Gebrauch einer Trocknung unterzogen werden. Nach der Trocknung liegt der Katalysator offenbarungsgemäß als Pulver mit Teilchengrößen im Bereich von 4 bis 6 nm vor, wobei das Ru im erhaltenen trockenen Pulver als Ru-(IV)-oxidhydrat mit ca. 50 Gew.-% Ru vorliegt.

In DE 101 19 135 A1 wird die kontinuierliche Herstellung von Bis-(4-aminocyclohexyl)-methan durch Hydrierung von Bis(4-aminophenyl)methan mit Hilfe eines pulverförmigen Ruthenium-Katalysators beschrieben. Das Ruthenium wurde dabei auf einem inerten Trägermaterial, z. B. Al₂O₃ aufgebracht und mit einer Partikelgröße von 5 -150 µm eingesetzt.

US 5,214,212 lehrt den Zusatz von Metall-Salzen als Promotoren in einem Verfahren zur Hydrierung von aromatischen Aminen. Die Zugabe der Promotoren führt offenbarungsgemäß zur Verbesserung der Reaktionsgeschwindigkeit und zur Reduzierung der Nebenproduktbildung. Die Metallsalze wurden in Konzentrationen von 0,3% bis 10%, bezogen auf das aromatische Amin, eingesetzt.

In US 3,864,361 wird die Herstellung von 2,5-Dimethlypyrrolidon durch Reduktion von 2,5-Dimethylpyrrol in Gegenwart von feinverteilten, ungeträgerten RuO₂beschrieben. Der Katalysator wird nach Beendigung der Hydrierung durch Filtration entfernt. Offenbarungsgemäß kann die Abtrennung des Ru-Katalysators durch die Zugabe von Al₂O₃ als Filtrierhilfsmittel verbessert werden.

Der Stand der Technik lehrt die Verwendung von heterogenen Ru-Katalysatoren mit kleinen Partikelgrößen, welche möglichst fein verteilt im Reaktionsgemisch vorliegen sollen. Werden solche Katalysatoren in einem großtechnischen Prozess eingesetzt zeigt sich jedoch, dass die kleinen Katalysatorpartikel während der Hydrierung größeren Agglomeraten bilden, die sich dann im Reaktor absetzen können. Dadurch wird zum einen ein Teil der Katalysatormenge dem Prozess entzogen, zum anderen kann es aufgrund der resultierenden Ablagerungen zu Verstopfungen kommen, die zu Störungen im Betriebsablauf führen.

In den aromatischen Ausgangsprodukten, die in das Verfahren zur Herstellung von cycloaliphatischen Aminen eingesetzt werden, befinden sich üblicherweise geringe Mengen an Nebenprodukten, die während der Reinigung der Ausgangsprodukte in der Regel nicht quantitativ entfernt werden können. Diese Nebenprodukte können den Ruthenium-Katalysator vergiften und dadurch die Raum-Zeit-Ausbeute des Verfahrens vermindern.

Im Falle der Hydrierung von Bis(4-aminophenyl)methan bzw. Bis(4-amino-3-methylphenyl)methan zu den jeweiligen Cyclohexylderivaten handeltes sich bei diesen Nebenprodukten hauptsächlich um die Hydrochloride der aromatischen Ausgangsamine und um höher siedende aromatische Produkte.
Bereits dann, wenn der Chlorgehalt der jeweiligen Ausgangsprodukte höher als 1 ppm liegt oder die Konzentration an höher siedenden Produkten den Wert 2 Gew.-% überschreitet, können sich beträchtliche Verluste bei der Raum-Zeit-Ausbeute ergeben.

Der vorliegenden Erfindung lag deshalb die Aufgabe zu Grunde, ein Verfahren zur Herstellung von cycloaliphatischen Verbindungen zur Verfügung zu stellen, welches eine hohe Raum-Zeit-Ausbeute aufweist. Zudem sollte ein Verfahren entwickelt werden, das einen stabilen, großtechnischen Betrieb gewährleistet und die Probleme, die durch Agglomeration von Katalysatorpartikel und den daraus resultierenden Verstopfungen auftreten, verringert. Weiterhin soll mit Hilfe des erfindungsgemäßen Verfahrens die Ausbeute an Produkt erhöht und die Bildung von Nebenprodukten verringert werden, insbesondere bei der Verwendung von Ausgangsstoffen, die einen gewissen Grad an störenden Nebenprodukten enthalten. Somit sollte ein insgesamt stabiler Produktionsbetrieb gesichert werden und eine hohe Ausbeute und Selektivität gewährleistet werden.

Erfindungsgemäß wurde die Aufgabe durch ein Verfahren zur Herstellung von cyclo aliphatischen Aminen durch Hydrierung der entsprechenden aromatischen Verbindungen mit Wasserstoff enthaltendem Gas bei einer Temperatur von 30 bis 280°C und einem Druck von 50 - 350 bar, in Gegenwart von Ru-haltigen Katalysatoren, dadurch gekennzeichnet, dass man die Hydrierung in Gegenwart von 1 Gew.-% bis 500 Gew.-%, bezogen auf den Katalysator (berechnet als elementares Ruthenium), suspendierter anorganischer Zusatzstoffe durchführt, und die anorganischen Zusatzstoffe oder Metallseifen eine spezifische Oberfläche von 10 bis 1000m²/g aufweisen, gelöst.

In das erfindungsgemäße Verfahren werden aromatische Verbindungen eingesetzt, die zu cycloaliphatischen Aminen hydriert werden können.

Aromatische Verbindungen, die zu cycloaliphatischen Aminen hydriert werden können, sind üblicherweise ein- oder mehrkernige aromatische Verbindungen, die einen oder mehrere stickstoffhaltige Substituenten enthalten.

In einer bevorzugten Ausführungsform werden aromatische Verbindungen mit einem oder mehreren stickstoffhaltigen Substituenten eingesetzt, bei denen das Stickstoffatom des stickstoffhaltigen Substituenten direkt am aromatischen Ringe gebunden ist (N-substituierte aromatische Verbindungen).

Vorzugsweise werden aromatische Mono-, Di- oder Polyamine eingesetzt, die zu den entsprechenden cycloaliphatischen Aminen hydriert werden können.

Als aromatische Amine kommen ein- oder mehrkernige aromatischen Verbindungen mit ein oder mehreren Amingruppen in Betracht, beispielsweise:
aromatische Monoamine, wie Anilin, die isomeren Toluidine, die isomeren Xylidine, 1- bzw. 2-Aminonaphthalin, Benzidin und substituierte Benzidine;
aromatische Diamine, wie die isomeren Phenylendiamine, die isomeren Tolylendiamine, die isomeren Diaminonaphthalene, 4,4'-Diamino-3,3'-dimethyl-diphenylmethan, 4,4'-Diamino-3,3',5,5'-tetramethyl-diphenylmethan und 4,4'-Diamino-diphenylmethan; oder
aromatische Polyamine, wie Polymer-MDA (Polymethylen-Polyphenyl-Amin).

Als aromatischen Verbindungen, die zu cycloaliphatischen Aminen hydriert werden können, können auch Aromaten mit Nitro-, Nitril und Urethangruppen als Substituenten am aromatischen Ring eingesetzt werden,
beispielsweise
aromatische Verbindungen mit Nitrogruppen als Substituenten, wie Nitrobenzol, Nitrotoluol, Dinitrobenzol, Dinitrotoluol und die isomeren Nitroaniline;
aromatische Verbindungen mit Nitrilgruppen als Substiutenten, wie Benzonitril, Toluonitril oder. o-Aminobenzonitril; oder
aromatische Verbindungen mit Urethangruppen als Substituenten, wie die Dialkylurethane, die aus 4,4'-Methylen-diphenyl-diisocyanat, 2,4'-Methylen-diphenyl-diisocyanat oder 2,2'-Methylen-diphenyl-diisocyanat und aliphatischen Alkoholen, wie C₁-C₆-Alkohole, insbesondere n-Butanol gebildet werden,
die Dialkylurethane, die aus Toluylen-2,4-diisocyanat oder Toluylen-2,6-diisocyanat und aliphatischen Alkoholen, wie C₁-C₆-Alkohole, insbesondere n-Butanol gebildet werden,
die Dialkylurethane, die aus polymeres Diphenylmethandüsocyanat und aliphatischen Alkoholen, wie C₁-C₆-Alkohole, insbesondere n-Butanol gebildet werden,
die Dialkylurethane, die aus 2,4-Phenylendiisocyanat oder 2,6-Phenylendiisocyanat und aliphatischen Alkoholen, wie C₁-C₆-Alkohole, insbesondere n-Butanol gebildet werden, oder
die Dialkylurethane, die aus 1,5-Naphthylendiidocyanat und aliphatischen Alkoholen, wie C₁-C₆-Alkohole, insbesondere n-Butanol gebildet werden.

Die aromatischen Verbindungen können zusätzlich zu den Substituenten, die zu Amingruppen hydriert werden können, keine weiteren Substituenten aufweisen oder sie können ein oder mehrere weitere Substituenten tragen, beispielsweise Alkyl-, Cycloalkyl-, Aryl-, Heteroaryl-, Halogen-, Halogenalkyl-, Silyl-, Hydroxy-, Alkoxy-, Aryloxy-, Carboxy- oder Alkoxycarbonyl-Substituenten.

Bevorzugt werden in das Verfahren aromatische Amine, wie die voranstehend genannten aromatischen Mono-, Di- und/oder Polyamine, eingesetzt.

Besonders bevorzugt wird Polymer-MDA, Anilin, 2,4-Diaminotoluol, 2,6-Diaminotoluol, o-Phenylendiamin, m-Phenylendiamin, p-Phenylendiamin, 4,4'-Diamino-3,3'-dimethyl-diphenylmethan, 4,4'-Diamino-3,3',5,5'-tetramethyl-diphenylmethan und/oder 4,4'-Diamino-diphenylmethan in das Verfahren eingesetzt.
Ganz besonders bevorzugt werden Anilin, 4,4'-Diamino-3,3'-dimethyl-diphenylmethan, 4,4'-Diamino-3,3',5,5'-tetramethyl-diphenylmethan und/oder 4,4'-Diamino-diphenylmethan verwendet.

In einer besonderen Ausführungsform, werden in das erfindungsgemäße Verfahren aromatische Verbindungen, die zu den entsprechenden cycloaliphatischen Aminen hydriert werden können, eingesetzt, die Nebenprodukte enthalten. Beispiele für solche Nebenprodukte sind Hydrochloride der aromatischen Ausgangsamine oder höher siedende aromatische Nebenprodukte. Als höher siedende Nebenprodukte werden solche Bestandteile bezeichnet, die einen höheren Siedepunkt aufweisen als die aromatischen Ausgangsverbindungen, die zu den cycloaliphatischen Produkten hydriert werden sollen.
Vorzugsweise beträgt der Chlor-Gehalt der aromatischen Verbindungen, die zu den entsprechenden cycloaliphatischen Aminen hydriert werden können, 1 ppm oder mehr, vorzugsweise 10 ppm bis 10000 ppm und besonders bevorzugt 20 ppm bis 1000 ppm, wobei der Chlor-Gehalt üblicherweise gemäß DIN V 51408 Teil 2 ermittelt wird.
Der Gehalt an höher siedenenden aromatischen Verbindungen in den Ausgangsverbindungen beträgt in der Regel 1 Gew.-% und mehr, bevorzugt 2 bis 20 Gew.-% und besonders bevorzugt 2 bis 10 Gew.-%, wobei der Gehalt an höher siedenden aromatischen Verbindungen durch Labordestillation entsprechend ASTM D 5236-03 bei einem Druck von 1 mbar und einer Temperatur bis 260°C ermittelt wird.

In das erfindungsgemäße Verfahren wird ein Wasserstoff enthaltendes Gas eingesetzt. Der Wasserstoff kommt im Allgemeinen technisch rein zum Einsatz. Der Wasserstoff kann auch in Form eines Wasserstoff enthaltenden Gases, d.h. in Beimengungen mit anderen Inertgasen, wie Stickstoff, Helium, Neon, Argon oder Kohlendioxid zum Einsatz kommen. Als Wasserstoff enthaltende Gase können beispielsweise Reformerabgase, Raffineriegase usw. verwendet werden, wenn und soweit diese Gase keine Kontaktgifte für die Ru-haltigen Katalysatoren, wie zum Beispiel CO enthalten. Bevorzugt wird jedoch reiner Wasserstoff bzw. im Wesentlichen reiner Wasserstoff in das Verfahren eingesetzt.

Die Herstellung der aromatischen Amine erfolgt in Gegenwart von Ru-haltigen Katalysatoren

In das erfindungsgemäße Verfahren zur Hydrierung von aromatischen Verbindungen wird in der Regel ein heterogener Ru-haltiger Katalysator eingesetzt.

Der in die Verfahren eingesetzte Ru-haltige Katalysator kann unterschiedliche Morphologien aufweisen.
So können Ru-haltige Katalysatoren Trägermaterialien enthalten.
Als Trägermaterial werden üblicherweise Kohlenstoff, wie Graphit, Russ und/oder Aktivkohle, Aluminiumoxid, Siliziumdioxid, Zirkoniumdioxid, Zeolithe, Alumosilicate, etc. sowie Mischungen aus diesen Trägermaterialien verwendet.

Geträgerte Katalysatoren können durch bekannte Verfahren, wie Imprägnierung bzw. Tränkung (z.B. beschrieben in A. B. Stiles, Catalyst Manufacture - Laboratory and Commercial Preperations, Marcel Dekker, New York, 1983) oder Auffällung (z.B. beschrieben in EP-A2-1106 600, Seite 4, und A. B. Stiles, Catalyst Manufacture, Marcel Dekker, Inc., 1983, Seite 15) hergestellt werden.
Hierbei kann die Form und Größe des Trägermaterials variieren. Typische mittlere Korngrößen von Trägermaterialien liegen im Bereich von 0,0001 bis zu 5 Millimetern, vorzugsweise 0,0005 bis 1 mm, besonders bevorzugt 0,001 bis 0,5 mm.
Der Ru-haltige Katalysator kann auch ohne Trägermaterial eingesetzt werden, beispielsweise kann Ru als RuO₂ oder Oxidhydraten des Rutheniums eingesetzt werden. Typischerweise liegt die mittlere Teilchengröße von heterogenen Ru-haltigen Katalysatoren, die durch Fällung erhalten wurden, im Bereich von 1 nm bis 1 µm, bevorzugt im Bereich von 3 nm bis 100 nm. Beispielsweise beträgt die mittlere Teilchengröße der Rutheniumpartikel, die als Oxidhydrate des Ru eingesetzt werden, gemäß der Offenbarung von DE-OS-2132546 zwischen 4 und 6 nm.
Als heterogener Ru-haltiger Katalysator wird bevorzugt ein Katalysator auf Basis von Ru-Oxidhydrat eingesetzt.
Besonders bevorzugt wird ein Ru-haltiger Katalysator eingesetzt, wie er in der DE-OS-2132547 offenbart ist. Die Herstellung solcher bevorzugter Katalysatoren ist beispielsweise auf den Seiten 4 bis 5 sowie in dem Beispiel 1 der DE-OS-2132547 näher beschrieben.

Die Ru-haltigen Katalysatoren werden üblicherweise in suspendierter Form eingesetzt.

Das erfindungsgemäße Verfahren wird in Gegenwart anorganischer Zusatzstoffe durchführt.

Die spezifische Oberfläche der anorganischen Zusatzstoffe, gemessen in Anlehnung an ISO 5794-1, Annex D, liegt im Bereich von 10 bis 1000 m²/g. Bevorzugt sind Stoffe, welche eine Oberfläche im Bereich von 20 bis 600 m²/g, besonders bevorzugt im Bereich von 25 bis 300 m²/g aufweisen.

Die mittlere Primärteilchengröße der anorganischen Zusatzstoffe liegt in der Regel im Bereich von 1 bis 500 nm, bevorzugt bei 5 bis 200 nm und besonders bevorzugt bei 5 bis 100 nm und wird üblicherweise mittels Elektronmikroskopie, z.B. TEM, ermittelt.

Die Korngröße (d50) der anorganischen Zusatzstoffe (Laserbeugung in Anlehnung an ISO 13320-1) liegt üblicherweise bei 1 bis 200 µm.

Die Stampfdichte der anorganischen Zusatzstoffe (gemessen in Anlehnung an ISO 787-11) liegt üblicherweise im Bereich von 10 bis 2000 g/l, vorzugsweise im Bereich von 50 bis 1000 g/l und besonders bevorzugt im Bereich von 50 bis 500 g/l.

Die anorganischen Zusatzstoffe werden im erfindungsgemäßen Verfahren in suspendierter Form eingesetzt.

Als anorganische Zusatzstoffe können beispielsweise anorganische Zusatzstoffe, ausgewählt aus der Gruppe bestehend aus Aluminiumoxid, Siliziumdioxid, Alkali-, Erdalkali- und Aluminiumsilikaten, Alkali-, Erdalkali- und Aluminiumborosilikaten, Titandioxid, Metallseifen, Zeolite, Mangnesiumoxid, Zinkoxid, Zirkoniumoxid und anorganische Kohlenstoffverbindungen in das erfindungsgemäße Verfahren eingesetzt werden.

Als anorganischer Zusatzstoff kann Aluminiumoxid eingesetzt werden, beispielsweise α-Aluminiumoxid oder γ-Aluminiumoxid. Besonders bevorzugt wird γ-Aluminiumoxid eingesetzt.

Die spezifische Oberfläche von γ-Aluminiumoxid liegt in der Regel im Bereich von 150 bis 400 m²/g (vgl. Ullmanns Enzyklopädie der Technischen Chemie, 4. Auflage, Band 7, S. 293 ff).

Als anorganischer Zusatzstoff kann auch Siliziumdioxid eingesetzt werden.

Beispiele für Siliziumdioxid, das in das erfindungsgemäße Verfahren eingesetzt werden kann, sind Kieselgur oder bestimmte Formen von Kieselsäuren (vgl. Ullmanns Enzyklopädie der Technischen Chemie, 4. Auflage, Band 21, S. 439 ff).

Als Kieselgur bezeichnet man üblicherweise Diatomeenerde (Diatomit), die aus abgelagerten Sedimenten von Kieselalgen entstanden ist. Aufbereitete Kieselgur weist im Allgemeinen eine spezifische Oberfläche von 10 bis 25 m²/g auf.

Die Kieselsäuren unterscheidet man in der Regel nach der Art ihrer Herstellung. Beispiel für Kieselsäuren sind pyrogene Kieselsäuren, Fällungskieselsäuren und chemisch nachbehandelte Kieselsäuren.

Unter der Bezeichnung pyrogene Kieselsäuren werden im Allgemeinen die hochdispersen Kieselsäuren zusammengefasst, die bei hohen Temperaturen durch Koagulation aus der Gasphase gewonnen werden.

Fällungskieselsäuren werden in der Regel durch Ausfällung aus einer wässrigen Wasserglaslösung mittels Säure (meist Schwefelsäure), sich anschließender Abtrennung des Niederschlags und dessen Trocknung bzw. Sprühtrocknung erhalten.

Unter chemisch nachbehandelten Kieselsäuren versteht man üblicherweise pyrogene Kieselsäuren oder Fällungskieselsäuren, deren Oberfläche durch Umsetzung z. B. mit Chlorsilanen oder Siliconsäuren chemisch verändert wurden. Beispiele für pyrogene oder Fällungskieselsäuren sind die Produkttypen "Sipernat® D17" sowie "Sipernat® 22" der Firma Evonik-Degussa GmbH.

Die spezifische Oberfläche der genannten Kieselsäuren liegt je nach Herstellungsprozess im Bereich von 10 bis 1000 m²/g. Ihre Primärteilchengröße beträgt ca. 5 bis 500 nm. Typische Kieselsäuren, die als Zusatzstoffe verwendet werden können, weisen beispielsweise folgende Werte auf:

| | Spezifische Oberfläche m²/g | Mittl. Primärteilchengröße nm | Stampfdichte ISO 787-11 g/l |
|---|---|---|---|
| Pyrogene Kieselsäuren | 25-600 | 5-500 | 50-200 |
| Fällungskieselsäuren | 30-1000 | 3-100 | 50-1000 |
| Chem. nachbehandelte Kieselsäuren | ca. 110 | ca. 28 | ca. 80 |

Als anorganische Zusatzstoffe können auch Alkali-, Erdalkali- und Aluminiumsilikate eingesetzt werden. Hierbei können sowohl natürlich vorkommende als auch synthetische Silikate eingesetzt werden.

Insbesondere sind als Aluminiumsilikate die Tonminerale Bentonit, Walkerde, Bolus, Umbra, Tonerde, Montmorillonit, Vermiculit oder Kaolin zu nennen. Diese natürlich vorkommenden Produkte enthalten teilweise in untergeordneten Mengen weitere Metallbestandteile, beispielsweise Eisen (vgl. Ullmanns Enzyklopädie der Technischen Chemie, 4. Auflage, Band 23, S. 311 ff).

Auch Calciumsilikate oder Natriumaluminiumsilikate können als anorganische Zusatzstoffe Verwendung finden. Die spezifische-Oberfläche-dieser-Silikate beträgt im Allgemeinen 20 bis 200 m²/g. Als synthetisches Natriumaluminiumsilikat ist insbesondere der Typ "Sipernat® 820A" (Firma Evonik-Degussa GmbH) mit einer spezifischen Oberfläche von 85 m²/g und einer d50-Teichengröße von 7,5 µm bevorzugt.

Als anorganische Zusatzstoffe sind weiterhin Alkali-, Erdalkali- und Aluminiumborosilikate zu nennen. Bei den Borosilikaten sind die SiO₄-Tetraeder, die den Silikaten als Bausteine zugrunde liegen, teilweise durch BO₄-Tetraeder ersetzt. Beispiele für Borosilikate sind Turmalin, Tonerde-Turmalin oder Magnesia-Tonerde-Turmalin. Die Werte für spezifische Oberfläche und Primärteilchengröße sind ähnlich den Werten, wie sie bei den Silikaten genannt wurden.

Als anorganischer Zusatzstoff kann auch Titandioxid verwendet werden. Titandioxid kann beispielsweise in der Anatas- oder der Rutil-Modifikation vorliegen. Bevorzugt wird Titandioxid in der Anatas-Modifikation eingesetzt.

Die spezifische Oberfläche von Titandioxid beträgt typischerweise ca. 30 bis 70 m²/g, die Primärteilchengröße liegt im Allgemeinen zwischen ca. 20 und 40 nm und die Stampfdichte beträgt in der Regel im Bereich von 120 bis 170 g/l.

Metallseifen können ebenfalls als anorganische Zusatzstoffe in das erfindungsgemäße Verfahren eingesetzt werden.
Beispiele für Metallseifen sind die Aluminium-, Cadmium-, Lithium-, Calcium-, Magnesium- oder Zinksalze von Fettsäuren, wie Myristinsäure, Palmitinsäure oder Stearinsäure. Die Korngröße der Metallsalze ist im Allgemeinen kleiner als 200 µm, vorzugsweise kleiner als 100 µm.

Es können auch natürlich vorkommende oder synthetische Zeolithe als anorganische Zusatzstoffe im erfindungsgemäßen Verfahren eingesetzt werden. Die spezifische Oberfläche der Zeolithe liegt in vielen Fällen bei 800 m²/g oder darüber. Die Größe der synthetisch erzeugten Zeolith-Teilchen liegt in der Regel im Bereich von 1 bis 100 µm.

Als weitere geeignete Zusatzstoffe sind Magnesiumoxid, Zinkoxid und Zirkoniumoxid zu nennen.

Als anorganische Zusatzstoffe können auch anorganische Kohlenstoffverbindungen im erfindungsgemäßen Verfahren eingesetzt werden. Anorganische Kohlenstoffverbindungen sind beispielsweise Aktivkohlen, Kohlenstoff-Molekularsiebe sowie natürlicher oder synthetischer Ruß. Die spezifische Oberfläche dieser Kohlenstoffverbindungen liegt je nach Typ und Herstellverfahren üblicherweise im Bereich von 800 -1000 m²/g bei Aktivkohlen, in der Regel bei <100 m²/g bei Kohlenstoff-Molekularsieben und üblicherweise bei < 400 m²/g bei Ruß.

Bevorzugt werden als anorganische Zusatzstoffe Aluminiumoxid, Siliziumdioxid, Alkali-, Erdalkali- und Aluminiumsilikate, Alkali-, Erdalkali- und Aluminiumborosilikate, Titandioxid, Metallseifen, Zeolithe, Magnesiumoxid, Zinkoxid, Zirkoniumoxid oder anorganische Kohlenstoffverbindungen eingesetzt.

Besonders bevorzugt werden als anorganische Zusatzstoffe Siliziumdioxid, Alkali-, Erdalkali- und Aluminiumsilikate, Alkali-, Erdalkali- und Aluminiumborosilikate, Titandioxid, Metallseifen, Zeolithe, Magnesiumoxid, Zinkoxid, Zirkoniumoxid oder anorganische Kohlenstoffverbindungen eingesetzt.

Ganz besonders bevorzugt werden als anorganische Zusatzstoffe Siliziumdioxid, Alkali-, Erdalkali- und Aluminiumsilikate oder Alkali-, Erdalkali- und Aluminiumborosilikate eingesetzt.

Insbesondere bevorzugt wird Siliziumdioxid in das erfindungsgemäße Verfahren eingesetzt.

Die genannten Zusatzstoffe sind, wie oben ausgeführt, alle bekannt und zum großen Teil handelsüblich. Sie finden üblicherweise Anwendung als Filtrierhilfsmittel, Adsorptionsmaterialien, als Trägermaterial für Katalysatoren oder als Katalysatoren.

Erfindungsgemäß werden die anorganischen Zusatzstoffe in einer Menge von 1 Gew.-% bis 500 Gew.-%, bezogen auf den Katalysator (berechnet als elementares Ruthenium), zugegeben. Vorzugsweise verwendet man 5 bis 200 Gew.-% und insbesondere 20 bis 120 Gew.-% an anorganischen Zusatzstoffen, jeweils bezogen auf den Katalysator (berechnet als elementares Ruthenium.

Die Menge der eingesetzten heterogenen Ru-haltigen Katalysatoren in den oben genannten Verfahren liegt üblicherweise in einem Bereich von 0,0005 bis 5 Gew.-% Ruthenium, als Metall berechnet, bezogen auf die umzusetzende Substanz, insbesondere auf die zu hydrierende Ausgangssubstanz. Vorzugsweise beiträgt die Menge der eingesetzten Ru-haltigen Katalysatoren im Bereich von 0,001 bis 1 Gew.-% Ruthenium bezogen auf die umzusetzende Substanz, insbesondere auf die zu hydrierende Ausgangssubstanz. Besonders bevorzugt beträgt die Menge der eingesetzten Ru-haltigen Katalysatoren im Bereich von 0,001 bis 0,1 Gew.-% Ruthenium bezogen auf die umzusetzende Substanz.

Die Hydrierung kann diskontinuierlich oder kontinuierlich durchgeführt werden.

Bei der diskontinuierlichen Reaktionsführung kann die Hydrierung beispielsweise in einem Rührkessel bzw. Rührautoklaven, einem Schlaufenreaktor, einem Strahlschlaufenreaktor, einer Blasensäule oder einem Reaktor mit Umpumpkreislauf durchgeführt werden. Bevorzugt wird die diskontinuierliche Hydrierung in einem Rührkessel bzw. Rührautoklaven durchgeführt.

Bei der kontinuierlichen Reaktionsführung wird die Hydrierung üblicherweise in einem kontinuierlich betriebenen Rührkesselreaktor, einem kontinuierlich betriebenen Schlaufenreaktor, einem kontinuierlich betriebenen Strahlschlaufenreaktor, einer kontinuierlich betriebenen Blasensäule oder einem kontinuierlich betriebenen Reaktor mit Umpumpkreislauf oder einer Rührkesselkaskade durchgeführt.

Das erfindungsgemäße Verfahren wird bei einem Druck von 50 - 350 bar durchgeführt, vorzugsweise wird ein Druck von 150 bis 250 bar angewendet.

Erfindungsgemäß wird das Verfahren bei einer Temperatur im Bereich zwischen 30 und 280°C durchgeführt, wobei der Temperaturbereich von 120 bis 260°C besonders bevorzugt wird.

Die Hydrierung kann mit oder ohne Lösungsmittel durchgeführt werden. Als Lösungsmittel werden Alkohole, wie Isopropanol, Isobutanol oder t-Butanol, oder Ether, wie Diethylether, Glykoldimethylether, Dioxan oder Tetrahydrofuran, verwendet.

Als Lösungsmittel kann aber auch das bei der Reaktion entstehende Endprodukt eingesetzt werden.

Als Lösungsmittel kommen auch Mischungen der voranstehend genannten Lösungsmittel in Betracht.

Bevorzugte Lösungsmittel sind Isopropanol, Isobutanol und/oder t-Butanol. Besonders bevorzugt wird als Lösungsmittel das bei der Reaktion entstehende Endprodukt eingesetzt.

Das Lösungsmittel wird gewöhnlich in einer solchen Menge angewendet, so dass man 10 bis 50 %ige (Gew.%), bevorzugt 15 bis 40%ige, besonders bevorzugt 20 bis 30%ige Lösungen der zur Hydrierung vorgesehenen aromatischen Verbindungen erhält.

Besonders vorteilhaft für die kontinuierliche Durchführung des Verfahrens ist es, das bei der Reaktion entstehende Endprodukt als Lösungsmittel anzuwenden.

Der Ru-haltige Katalysator und der anorganische Zusatzstoff werden als Suspension des Katalysators und des Zusatzstoffes in den eingesetzten flüssigen Edukten bzw. Lösungsmitteln eingesetzt.

Bei diskontinuierlicher Arbeitsweise wird der Ru-Katalysator üblicherweise, entweder als trockenes Pulver oder als wasserfeuchter Filterkuchen, zusammen mit dem anorganischen Zusatzstoff direkt in den Hydrierreaktor gegeben.

Besonders vorteilhaft wird der Ru-Katalysator gemeinsam mit dem anorganischen Zusatzstoff mit einem Lösungsmittel, dem flüssigen Einsatzstoff oder flüssigem Reaktionsaustrag zu einer Suspension vermischt, welche dann mittels geeigneter Dosierpumpen dem Reaktor zugeführt werden kann. Bei kontinuierlicher Arbeitsweise wird diese Katalysator-Suspension üblicherweise dem Hydrierreaktor kontinuierlich zugeführt.
Einige anorganische Zusatzstoffe, z. B. Kieselgur oder Fällungskieselsäuren können auch bereits bei der Fällung der Ru-(IV)-oxidhydrat-Partikel anwesend sein, d. h. man kann sie vor Ausfällung des Rutheniumoxidhydrats zur jeweiligen wässrigen Rutheniumsalzlösung hinzufügen. Ihre Zugabe zur Reaktionsmischung erfolgt dann direkt zusammen mit dem Katalysator.

Das Reaktionsgemisch aus der Hydrierung wird üblicherweise aufgereinigt.
Die Aufreinigung des Reaktionsgemisches erfolgt üblicherweise durch Rektifikation bzw. Destillation.
Der anorganische Zusatzstoff sowie der heterogene Ru-haltige Katalysator können vor der Destillation entfernt werden, beispielsweise durch eine Fest-Flüssig-Trennung, wie Filtration, Sedimentation oder Zentrifugation.
Lösungsmittel und nicht umgesetzte Ausgangsstoffe können in das Verfahren zurückgeführt werden.

Die durch das erfindungsgemäße Verfahren erhältlichen cycloaliphatischen Amine können als Synthesebaustein für die Herstellung von Tensiden, Arznei- und Pflanzenschutzmitteln, Stabilisatoren, Lichtschutzmitteln, Polymeren, Isocyanaten, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern, Emulgatoren und/oder als Ausgangssubstanzen für die Herstellung von Harnstoffen und Polyharnstoffen verwendet werden.
Insbesondere kann Cyclohexylamin, erhältlich durch die Hydrierung von Anilin, als Korrosionsinhibitor oder Vulkanisationsbeschleuniger verwendet werden. 4,4'-Diamino-dicyclohexylmethan, 4,4'-Diamino-3,3',5,5'-tetramethyl-dicyclohexylmethan oder 4,4'-Diamino-3,3'-dimethyl-dicyclohexylmethan können als Monomerbaustein für Polyamide, als Härter für Epoxyharze oder als Ausgangsprodukt für die Herstellung der entsprechenden Isocyanate verwendet werden.

-Die Vorteile der erfindungsgemäßen Verfahren bestehen darin, dass das erfindungsgemäße Verfahren in der Regel eine hohe Raum-Zeit-Ausbeute aufweist. Zudem gewährleistet das Verfahren üblicherweise einen stabilen Betrieb, da Probleme, die durch Agglomeration von Katalysatorpartikel und den daraus resultierenden Verstopfungen auftreten, verringert werden.
Durch die verringerte Ablagerungstendenz der Katalysatorpartikel wird im Allgemeinen gewährleistet, dass dem Reaktionsgemisch im Wesentlichen die gesamte eingesetzte Katalysatormenge zur Verfügung steht und nicht ein Teil des Katalysators durch Ablagerungen entzogen wird.
Weiterhin kann mit dem erfindungsgemäßen Verfahrens die Ausbeute an Produkt erhöht und die Bildung von Nebenprodukten verringert werden, insbesondere wenn Ausgangsstoffe verwendet werden, die einen gewissen Grad an störenden Nebenprodukten enthalten. Deshalb eignet sich das Verfahren in der Regel besonders für die Hydrierung von aromatischen Ausgangsstoffen, die Nebenprodukte, wie Hydrochloride der aromatischen Ausgangsamine oder höher siedende Verunreinigungen, enthalten.
Das erfindungsgemäße Verfahren ist in der Regel deshalb auch für Ausgangsprodukte geeignet, die ein Chlor-Gehalt von 1 ppm und mehr oder einen Anteil von höher siedenden Produkten aufweist, der 1 Gew.-% oder mehr beträgt.

Das erfindungsgemäße Verfahren wird durch die nachfolgenden Beispiele näher erläutert.

### Beispiele 1 bis 4

Bei den Beispielen 1 - 4 werden die Vorteile der vorliegenden Erfindung beim Einsatz einer chloridhaltigen Ausgangsverbindung dargelegt. Die Beispiele 1 bis 4 wurden jeweils unter gleichen Reaktionsbedingungen durchgeführt.

In Beispiel 1 wurde kein anorganischer Zusatzstoff zugegeben.

In den Beispielen 2 bis 4 wurden jeweils 5 Gew.-% bezogen auf den Katalysator (berechnet als elementares Ruthenium), anorganische Zusatzstoffe zugegeben. Dabei handelt es sich um folgende Komponenten:

### Beispiel 2: Metallseife Calciumstearat (Korngröße < 200 µm)

Beispiel 3: Hydrophile, kolloidale Fällungskieselsäure - sprühgetrocknet (spez. Oberfläche ca. 190 m²/g, mittlere Primärteilchengröße ca. 18 nm, Korngröße nach ISO 13320-1: 30 bis 200 µm) (Sipernat® 22 der Fa. Evonik-Degussa GmbH)

Beispiel 4: Hydrophobe, kolloidale chemisch nachbehandelte Kieselsäure (spez. Oberfläche ca. 110 m²/g, mittlere Primärteilchengröße ca. 28 nm) (Sipernat® D17 der Fa. Evonik-Degussa GmbH)

### Durchführung: Versuche 1 - 4

In einem Rollautoklaven mit Glaseinsatz wurden 250 Gewichtsteile Bis(4-amino-3 methylphenyl)methan, 2,5 Gewichtsteile Katalysatorsuspension und gegebenenfalls die jeweilige Menge an anorganischem Zusatzstoff (Beispiele 2 bis 4) vorgelegt. Die Katalysatorsuspension enthielt dabei 2 Gew.-%, bezogen auf die Suspension, Ruthenium-Katalysator (berechnet als elementares Ruthenium) und 0,134 Gew.-%, bezogen auf die Suspension, Bis(4-amino-3-methyl- phenyl)methan-hydrochlorid.

Der Autoklav wurde zunächst mit gasförmigem Stickstoff gespült, anschließend wurde in kaltem Zustand Wasserstoff eingepresst, bis sich ein Druck von 100 bar einstellte. Dann wurde mit dem Rollbetrieb begonnen und gleichzeitig innerhalb von 4 Stunden die Temperatur von Raumtemperatur auf 230°C erhöht. Nun wurde noch zusätzlicher Wasserstoff eingepresst, bis sich ein Druck von 270 bar einstellte.

Der Rollautoklav wurde dann 5 Stunden bei einer Temperatur von 230°C betrieben, wobei in stündlichem Abstand zusätzlicher Wasserstoff eingepresst wurde um den Druck von 270 bar aufrecht zu erhalten.

Danach wurde der Druck nochmals auf 270 bar Wasserstoff eingestellt und sowohl Rollantrieb als auch Heizung abgestellt.

Nachdem das Reaktionsgemisch Raumtemperatur erreicht hatte, wurde der Autoklav entspannt, das Reaktionsgemisch entnommen und vom Katalysator bzw. anorganischen Zusatzstoff abfiltriert.

Das Filtrat wurde dann einer fraktionierten Destillation unter vermindertem Druck unterworfen.

Die jeweils erhaltenen Ergebnisse sind nachfolgend wiedergegeben (Konzentrationsangaben in Gew.-%):

| | Beispiel 1 (Vergleich, ohne Zusatz) | Beispiel 2 Ca- Stearat | Beispiel 3 SiO₂ (hydrophil) | Beispiel 4 SiO₂ (hydrophob) |
|---|---|---|---|---|
| Leichtsiedende Nebenprodukte | 1,71 | 2,38 | 2,46 | 3,0 |
| Endprodukt | 47,19 | 68,7 | 75,14 | 71,58 |
| Zwischenstufe | 41,86 | 19,16 | 12,99 | 16,82 |
| Ausgangsprodukt | 0,24 | 0,16 | 0 | 0 |
| Hochsiedende Nebenprodukte | 9,0 | 9,6 | 9,4 | 8,6 |

### Endprodukt: Bis(4-amino-3-methylcyclohexyl)methan

### Zwischenstufe: (4-Amino-3-methylcyclohexyl)-(4-amino-3-methylphenyl)-methan

### Ausgangsprodukt: Bis(4-amino-3-methylphenyl)methan

Bei den Beispielen 2-4 konnten im Vergleich zu Beispiel 1 deutlich höhere Umsätze erzielt werden.

### Beispiel 5:

In Beispiel 5 wird eine kontinuierlich durchgeführte Hydrierung entsprechend dem erfindungsgemäßen Verfahren dargelegt.

Die Herstellung des Ru-(IV)-oxidhydrates wurde entsprechend Beispiel 1 in DE 21 32 547 durchgeführt, wobei lediglich auf die abschließende Trocknung im Vakuum verzichtet wurde. Zur Zubereitung der Katalysator-Suspension wurden 5500 g des so hergestellten Filterkuchens (mit 579 g Ru berechnet 100%) sowie 280 g hydrophobes SiO₂ (Sipernat D17^{®} der Fa. Evonik-Degussa GmbH) in einen Rührbehälter gegeben und mit 150 kg einer flüssigen Produktmischung (Austrag des Hydrierreaktors) intensiv vermischt, so dass die erzeugte Suspension 0,37 Gew.-% Ru enthielt.

Die Durchführung der Hydrierung wurde in einer kontinuierlich betriebenen Produktionsanlage mit einem gekühlten Hochdruck-Blasensäulenreaktor durchgeführt. In diesen Reaktor wurden kontinuierlich 500 kg/h einer flüssigen Schmelze von Bis(4-aminophenyl)methan sowie 6,7 kg/h der Katalysatorsuspension zugeführt. Der Druck im Reaktor wurde durch Nachführung von reinem Wasserstoff auf 200 bar geregelt. Die Temperatur im Reaktor wurde durch Kühlung im Bereich von 230 bis 240 °C konstant gehalten.

Das aus dem Reaktor austretende Produktgemisch wurde entspannt und auf eine Temperatur von 100 °C abgekühlt. Die Analyse die Produktmischung mittels Gaschromatographie ergab folgende Werte (Konzentrationsangaben in Gew.-%):

| | Analyse Reaktionsaustrag Gew.-% |
|---|---|
| Leichtsiedende Nebenprodukte | 7,85 |
| Hochsiedende Nebenprodukte | 2 |
| Endprodukt | 90 |
| Zwischenstufe | 0,1 |
| Ausgangsprodukt | 0,05 |

Endprodukt: Bis-(4-amino-cyclohexyl)-methan
Zwischenstufe: (4-Amino-cyclohexyl)-(4-amino-phenyl)-methan
Ausgangsprodukt: Bis(4-amino-phenyl)methan
Die Hydrierung konnte über einen Zeitraum von vier Wochen mit konstanten Bedingungen betrieben werden, ohne dass ein Nachlassen der Reaktivität oder aber ein Verstopfen des Reaktors durch Feststoffablagerungen beobachtet wurde.
Ohne Zusatz der anorganischen Zusatzstoffe treten Ablagerungen, die den Produktionsbetrieb stören und zu einer Verringerung der Ausbeute und Selektivität führen im Allgemeinen schon nach einer Woche auf.

## Patentansprüche

1. Verfahren zur Herstellung von cycloaliphatischen Aminen durch Hydrierung der entsprechenden aromatischen Verbindungen mit Wasserstoff enthaltendem Gas bei einer Temperatur von 30 bis 280°C und einem Druck von 50 - 350 bar, in Gegenwart von Ruthenium-haltigen Katalysatoren, **dadurch gekennzeichnet, dass** man die Hydrierung in Gegenwart von 1 Gew.-% bis 500 Gew.-%, bezogen auf den Katalysator (berechnet als elementares Ruthenium (Ru)), suspendierter anorganischer Zusatzstoffe oder Metallseifen durchführt und die anorganischen Zusatzstoffe oder Metallseifen eine spezifische Oberfläche von 10 bis 1000 m²/g aufweisen.

2. Verfahren gemäß Anspruch 1, **dadurch gekennzeichnet, dass** man als anorganische Zusatzstoffe, Stoffe, ausgewählt aus der Gruppe, bestehend aus Aluminiumoxid, Siliziumdioxid, Alkali-, Erdalkali- und Aluminiumsilikaten, Alkali-, Erdalkali- und Aluminiumborosilikaten, Titandioxid, , Zeolithe, Magnesium-oxid, Zinkoxid, Zirkoniumoxid und anorganische Kohlenstoffverbindungen, verwendet.

3. Verfahren gemäß mindestens einem der Anspruch 1 bis 2, **dadurch gekennzeichnet, dass** die anorganischen Zusatzstoffe oder Metallseifen eine mittlere Primärteilchengröße von 1 bis 500 nm aufweisen.

4. Verfahren gemäß mindestens einem der Ansprüche 1 bis 3, **dadurch gekennzeichnet, dass** als anorganische Zusatzstoffe hydrophobe Silikate verwendet werden.

5. Verfahren gemäß mindestens einem der Ansprüche 1 bis 4, **dadurch gekennzeichnet, dass** man als aromatische Verbindungen ein- oder mehrkernige aromatische Amine einsetzt.

6. Verfahren gemäß mindestens einer der Ansprüche 1 bis 5, **dadurch gekennzeichnet, dass** man die Hydrierung in kontinuierlicher Betriebsweise durchführt.

7. Verfahren gemäß mindestens einem der Ansprüche 1 bis 6, **dadurch gekennzeichnet, dass** man die Hydrierung in Anwesenheit eines Lösungsmittels durchführt.

8. Verfahren gemäß mindestens einem der Ansprüche 1 bis 7, **dadurch gekennzeichnet, dass** man das bei der Reaktion entstehende Endprodukt als Lösungsmittel einsetzt.

9. Verfahren gemäß mindestens einem der Ansprüche 1 bis 8, **dadurch gekennzeichnet, dass** man die Hydrierung in Gegenwart eines Ru-haltigen Katalysators durchführt, dessen Vorstufe ein Rutheniumoxidhydrat ist.

10. Verfahren gemäß mindestens einem der Ansprüche 1 bis 9, **dadurch gekennzeichnet, dass** man die Hydrierung in Gegenwart von 5 Gew.-% bis 200 Gew.-%, bezogen auf den Katalysator (berechnet als elementares Ruthenium), suspendierter anorganischer Zusatzstoffe durchführt.

11. Verfahren gemäß mindestens einem der Ansprüche 1 bis 10, **dadurch gekennzeichnet, dass** die in das Verfahren eingesetzten aromatischen Ausgangsverbindungen einen Chlorgehalt von 1 ppm oder mehr und/oder einen Gehalt an höher siedenden aromatischen Nebenprodukten von 1 Gew.-% oder mehr aufweisen.

12. Verfahren gemäß mindestens einem der Ansprüche 1 bis 11, **dadurch gekennzeichnet, dass** man als aromatische Verbindung Polymer-MDA, Anilin, 4,4'-Diamino-3,3'-dimethyl-diphenylmethan, 4,4'-Diamino-3,3',5,5'-tetramethyl-diphenylmethan und/oder 4,4'-Diamino-diphenylmethan einsetzt.

13. Verfahren zur Herstellung von Tensiden, Arznei- und Pflanzenschutzmitteln, Stabilisatoren, Lichtschutzmitteln, Polymeren, Isocyanaten, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, lonenaustauschern, Textilhilfsmitteln, Farbstoffen, Vulkanisationsbeschleunigern, Emulgatoren, Harnstoffen oder Polyharnstoffen durch Umsetzung eines cycloaliphatischen Amins, **dadurch gekennzeichnet, dass** in einer ersten Stufe das cycloaliphatisches Amin gemäß mindestens einem der Ansprüche 1 bis 12 hergestellt wird und anschließend in einer zweiten Stufe das cycloaliphatische Amin aus der ersten Stufe umgesetzt wird.

## Claims

1. A process for preparing cycloaliphatic amines by hydrogenating the corresponding aromatic compounds with hydrogen-comprising gas at a temperature of from 30 to 280°C and a pressure of 50 - 350 bar, in the presence of ruthenium catalysts, which comprises performing the hydrogenation in the presence of from 1% by weight to 500% by weight, based on the catalyst (calculated as elemental ruthenium (Ru)), of suspended inorganic additives or metal soaps and wherein the inorganic additives or metal soaps have a specific surface area of from 10 to 1000 m²/g.

2. The process according to claim 1, wherein the inorganic additives used are substances selected from the group consisting of aluminum oxide, silicon dioxide, alkali metal, alkaline earth metal and aluminum silicates, alkali metal, alkaline earth metal and aluminum borosilicates, titanium dioxide, zeolites, magnesium oxide, zinc oxide, zirconium oxide and inorganic carbon compounds.

3. The process according to at least one of claims 1 to 2, wherein the inorganic additives or metal soaps have a mean primary particle size of from 1 to 500 nm.

4. The process according to at least one of claims 1 to 3, wherein the inorganic additives used are hydrophobic silicates.

5. The process according to at least one of claims 1 to 4, wherein the aromatic compounds used are mono- or polycyclic aromatic amines.

6. The process according to at least one of claims 1 to 5, wherein the hydrogenation is performed in continuous mode.

7. The process according to at least one of claims 1 to 6, wherein the hydrogenation is performed in the presence of a solvent.

8. The process according to at least one of claims 1 to 7, wherein the end product formed in the reaction is used as the solvent.

9. The process according to at least one of claims 1 to 8, wherein the hydrogenation is performed in the presence of an Ru catalyst whose precursor is ruthenium oxide hydrate.

10. The process according to at least one of claims 1 to 9, wherein the hydrogenation is performed in the presence of from 5% by weight to 200% by weight, based on the catalyst (calculated as elemental ruthenium) of suspended inorganic additives.

11. The process according to at least one of claims 1 to 10, wherein the aromatic starting compounds used in the process have a chlorine content of 1 ppm or more and/or a content of higher-boiling aromatic by-products of 1% by weight or more.

12. The process according to at least one of claims 1 to 11, wherein the aromatic compound used is polymeric MDA, aniline, 4,4'-diamino-3,3'-dimethyldiphenyl-methane, 4,4'-diamino-3,3',5,5'-tetramethyldiphenylmethane and/or 4,4'-diaminodiphenylmethane.

13. A process for producing surfactants, pharmaceutical and crop protection compositions, stabilizers including light stabilizers, polymers, isocyanates, hardeners for epoxy resins, catalysts for polyurethanes, intermediates for preparing quaternary ammonium compounds, plasticizers, corrosion inhibitors, synthetic resins, ion exchangers, textile assistants, dyes, vulcanization accelerants, emulsifiers, ureas or polyureas by converting a cycloaliphatic amine, which comprises, in a first stage, preparing the cycloaliphatic amine according to at least one of claims 1 to 12 and subsequently, in a second stage, converting the cycloaliphatic amine from the first stage.

## Revendications

1. Procédé pour la préparation d'amines cycloaliphatiques par hydrogénation des composés aromatiques correspondants par un gaz contenant de l'hydrogène à une température de 30 à 280°C et une pression de 50-350 bars, en présence de catalyseurs contenant du ruthénium, **caractérisé en ce qu'**on réalise l'hydrogénation en présence de 1% en poids à 500% en poids, par rapport au catalyseur (calculé sous forme de ruthénium élémentaire (Ru)), d'additifs inorganiques ou de savons métalliques en suspension et les additifs inorganiques ou les savons métalliques présentent une surface spécifique de 10 à 1000 m²/g.

2. Procédé selon la revendication 1, **caractérisé en ce qu'**on utilise comme additifs inorganiques des substances choisies dans le groupe constitué par l'oxyde d'aluminium, le dioxyde de silicium, les silicates de métal alcalin, alcalino-terreux et d'aluminium, les borosilicates de métal alcalin, alcalino-terreux et d'aluminium, le dioxyde de titane, les zéolithes, l'oxyde de magnésium, l'oxyde de zinc, l'oxyde de zirconium et les composés carbonés inorganiques.

3. Procédé selon au moins l'une quelconque des revendications 1 à 2, **caractérisé en ce que** les additifs inorganiques ou les savons métalliques présentent une grosseur moyenne de particule primaire de 1 à 500 nm.

4. Procédé selon au moins l'une quelconque des revendications 1 à 3, **caractérisé en ce qu'**on utilise, comme additifs inorganiques, des silicates hydrophobes.

5. Procédé selon au moins l'une quelconque des revendications 1 à 4, **caractérisé en ce qu'**on utilise, comme composés aromatiques, des amines aromatiques à un ou plusieurs noyaux.

6. Procédé selon au moins l'une quelconque des revendications 1 à 5, **caractérisé en ce qu'**on réalise l'hydrogénation dans un mode opératoire continu.

7. Procédé selon au moins l'une quelconque des revendications 1 à 6, **caractérisé en ce qu'**on réalise l'hydrogénation en présence d'un solvant.

8. Procédé selon au moins l'une quelconque des revendications 1 à 7, **caractérisé en ce qu'**on utilise le produit final formé lors de la réaction comme solvant.

9. Procédé selon au moins l'une quelconque des revendications 1 à 8, **caractérisé en ce qu'**on réalise l'hydrogénation en présence d'un catalyseur contenant Ru, dont le précurseur est un oxyde de ruthénium hydraté.

10. Procédé selon au moins l'une quelconque des revendications 1 à 9, **caractérisé en ce qu'**on réalise l'hydrogénation en présence de 5% en poids à 200% en poids, par rapport au catalyseur (calculé sous forme de ruthénium élémentaire) d'additifs inorganiques en suspension.

11. Procédé selon au moins l'une quelconque des revendications 1 à 10, **caractérisé en ce que** les composés de départ aromatiques utilisés dans le procédé présentent une teneur en chlore de 1 ppm ou plus et/ou une teneur en produits secondaires aromatiques à point d'ébullition plus élevé de 1% en poids ou plus.

12. Procédé selon au moins l'une quelconque des revendications 1 à 11, **caractérisé en ce qu'**on utilise, comme composé aromatique, du MDA polymère, de l'aniline, du 4,4'-diamino-3,3'-diméthyldiphénylméthane, du 4,4'-diamino-3,3',5,5'-tétraméthyl-diphénylméthane et/ou du 4,4'-diaminodiphénylméthane.

13. Procédé pour la préparation d'agents tensioactifs, de médicaments et d'agents de phytoprotection, de stabilisants, d'agents de protection contre la lumière, de polymères, d'isocyanates, de durcisseurs pour les résines époxy, de catalyseurs pour les polyuréthanes, de produits intermédiaires pour la préparation de composés d'ammonium quaternaire, de plastifiants, d'inhibiteurs de corrosion, de résines synthétiques, d'échangeurs d'ions, d'adjuvants pour textiles, de colorants, d'accélérateurs de vulcanisation, d'émulsifiants, d'urées ou de polyurées par transformation d'une amine cycloaliphatique, **caractérisé en ce qu'**on prépare, dans une première étape, l'amine cycloaliphatique selon au moins l'une quelconque des revendications 1 à 12, puis on transforme dans une deuxième étape l'amine cycloaliphatique de la première étape.
